# EUROPÄISCHE PATENTSCHRIFT

(11) **EP 1 324 826 B1**
(45) Veröffentlichungstag und Bekanntmachung des Hinweises auf die Patenterteilung: **01.09.2004**
(21) Anmeldenummer: 01982272.5
(22) Anmeldetag: 11.09.2001
(51) Int. Cl.: C07D 301/12

(54) **VERFAHREN ZUR OXIDATION EINES ALKENS**
PROCESS FOR OXIDATION OF AN ALKYLENE
PROCEDE D'OXYDATION D'UNE ALKYLENE

(30) Priorität: 11.09.2000 DE 10044788
(43) Veröffentlichungstag der Anmeldung: 09.07.2003
(73) Patentinhaber: BASF AKTIENGESELLSCHAFT, 67056 Ludwigshafen (DE)
(72) Erfinder: MÜLLER, Ulrich, 67435 Neustadt (DE); TELES, Joaquim, Henrique, 67166 Otterstadt (DE); BERG, Anne, B-2170 Merksem (BE); HARDER, Wolfgang, 69469 Weinheim (DE); RUDOLF, Peter, 68526 Ladenburg (DE); REHFINGER, Alwin, 67112 Mutterstadt (DE); BASSLER, Peter, 68519 Viernheim (DE); RIEBER, Norbert, 68259 Mannheim (DE)
(74) Vertreter: Isenbruck, Günter, Dr.
(86) Internationale Anmeldenummer: PCT/EP2001/010489
(87) Internationale Veröffentlichungsnummer: WO 2002/022260

(56) Entgegenhaltungen:
- EP-A- 0 085 234
- WO-A-98/00413
- DE-A- 19 723 950

## Beschreibung

Die vorliegende Erfindung betrifft ein integriertes Verfahren zur Herstellung eines Epoxides.

Aus dem Stand der Technik ist bekannt, dass die katalytische Aktivität heterogener Katalysatoren für die Oxidation von organischen Verbindungen in der Flüssigphase, wobei hier insbesondere die Epoxidation von organischen Verbindungen mit mindestens einer C-C-Doppelbindung unter Verwendung eines Hydroperoxides in Gegenwart eines Zeolith-Katalysators von Bedeutung ist, mit fortschreitender Versuchszeit abnimmt und die entsprechenden Katalysatoren dann regeneriert werden müssen.

Demgemäß sind Verfahren zur Regenerierung von Zeolith-Katalysatoren bereits aus dem Stand der Technik bekannt. Hierzu verweisen wir auf die WO 98/55228 und den darin zitierten Stand der Technik. Im Rahmen dieses Standes der Technik werden grundsätzlich zwei verschiedene Vorgehensweisen zur Katalysatorregenerierung vorgeschlagen.
1. Sofern der Katalysator in Suspension eingesetzt wird, wird er zunächst vom flüssigen Reaktionsauftrag abgetrennt und in eine für die Regenerierung geeignete Regeneriereinrichtung überführt und dort durch thermische Behandlung in Anwesenheit von Sauerstoff regeneriert;
2. Wird der Katalysator als Festbett eingesetzt, wird die flüssige Phase abgelassen oder abgepumpt und der Katalysator entweder im Reaktor selbst oder in einer von diesem verschiedenen Regeneriereinrichtung durch thermische Behandlung in Abwesenheit von Sauerstoff regeneriert.

Darüber hinaus wurde im Stand der Technik bereits mehrfach eine Regenerierung durch Behandlung des Katalysators mit einer Flüssigkeit, die wiederum ein Oxidationsmittel, wie z.B. Wasserstoffperoxid, ist, bei erhöhter Temperatur beschrieben. Diesbezüglich verweisen wir auf DE-A 195 28 220 sowie die WO 98/18555.

Die EP-A 0-085 234 beschreibt ein Verfahren zur selektiven Umsetzung von Methanol und/oder Dimethylether in Kohlenwasserstoffen, insbesondere leichte Olefine wie C2-C3-Olefine. Zu dieser Umsetzung wird bei Temperaturen von 200 bis 500°C, einem Druck von 440 bis 3550 kPa und einer Weight Hourly Space Velocity der organischen Reaktanden von 0,05 bis 30 ein Katalysator eingesetzt, der ein kristallines Alumosilikat-Zeolith-Material umfasst. Zur Regenerierung des Katalysators wird dieser mit einem Wasserstoff enthaltenden Gas bei einer Temperatur von 200 bis 600°C und einem Druck von 440 bis 4930 kPa in Kontakt gebracht. Als Regeneriergas werden Wasserstoff und ein Gemisch aus Wasserstoff und Kohlenmonoxid beschrieben.

Angesichts dieses Stands der Technik lag der vorliegenden Erfindung die Aufgabe zugrunde, ein weiter verbessertes, insbesondere effektiveres Verfahren zur Regenerierung von Zeolith-Katalysatoren bereitzustellen, das sich problemlos in kontinuierliche und integrierte Verfahren zur Herstellung von Epoxiden der hier in Rede stehenden Art integrieren läßt, und das insbesondere ohne lange Abstell- und Ausfallzeiten zur Öffnung oder zum Umschluß der Reaktoren führt. Insbesondere sollte sich dieses Verfahren zur Regenerierung von Zeolith-Katalysatoren eignen, die bei einer Oxidation in Festbettfahrweise eingesetzt werden. Insbesondere sollte im Rahmen des erfindungsgemäßen Regenerierungssverfahrens die Ausbildung eines Hot-Spots in einem Katalysatorbett während der Regenerierung weitgehend verhindert oder zumindest stark abgeschwächt werden, da derartige lokale Überhitzungen des Katalysators sich nachteilig auf dessen Aktivität oder mechanischen Stabilität bei einem nachfolgenden erneuten Einsatz im erfindungsgemäßen integrierten Verfahren zur Oxidation eines Alkens auswirken.

Diese und weitere Aufgaben werden durch das erfindungsgemäße Verfahren zur Oxidation eines Alkens, das ein Verfahren zur Regenerierung eines Zeolith-Katalysators umfasst, gelöst.

Somit betrifft die vorliegende Erfindung ein Verfahren zur Oxidation eines Alkens, das umfasst: Umsetzung des Alkens mit einem Hydroperoxid in Gegenwart eines Zeolith-Katalysators und anschließende Regenerierung des Katalysators mittels eines Verfahrens, das eine thermische Behandlung des Katalysators in Gegenwart eines Gasstroms bei Temperaturen oberhalb von 120°C umfaßt, wobei der Gasstrom Wasserstoff enthält, und wobei der regenerierte Katalysator wieder zur Umsetzung des Alkens mit dem Hydroperoxid eingesetzt wird.

Dabei wird die Regenerierzeit vorzugsweise so gewählt, dass der Katalysator mindestens 85% seiner ursprünglichen Aktivität wieder erreicht.

Im Rahmen des erfindungsgemäßen Verfahrens können sowohl Katalysatoren in Pulverform, die als Suspension verwendet werden, als auch in einem Festbett gepackte Katalysatoren in Form eines Formkörpers, wie z.B. als Strang oder Extrudat, sowie als Netze, wie z.B. Edelstahl, Kanthal, oder Packungen kristallisierter Katalysatoren und Schalenkatalysatoren, bestehend aus einem inerten Kern aus SiO₂, α-Al₂O₃, hochkalziniertem TiO₂, Steatit und einer aktiven Katalysatorhülle, die einen Zeolith umfaßt, regeneriert werden.

Sofern der Katalysator in Suspensionsfahrweise verwendet wurde, muß er zunächst durch einen Abtrennschritt, wie z.B. Filtration oder Zentrifugieren von der Reaktionslösung abgetrennt werden. Der so gewonnene, zumindest teilweise deaktivierte pulverförmige Katalysator kann dann der Regenerierung zugeführt werden. Die während des Regenerierungsverfahrens bei erhöhten Temperaturen durchgeführten Stufen werden bei derartigen pulverförmigen Katalysatoren vorzugsweise in Drehrohröfen durchgeführt. Bei der Regenerierung eines Katalysators, der in Suspensionsfahrweise verwendet wird, ist es besonders bevorzugt, im Rahmen einer Kopplung der Umsetzung in Suspensionsfahrweise und des erfindungsgemäßen Regenerierungsverfahrens kontinuierlich einen Teil des zumindest teilweise deaktivierten Katalysators aus der Umsetzung zu entfernen, extern mittels des erfindungsgemäßen Verfahrens zu regenerieren und den regenerierten Katalysator wieder in die Umsetzung in Suspensionsfahrweise einzuschleusen.

Neben der Regenerierung von Katalysatoren in Pulverform können mit dem erfindungsgemäßen Verfahren auch Katalysatoren als Formkörper, beispielsweise solche, die in einem Festbett gepackt sind, regeneriert werden. Bei der Regenerierung eines im Festbett gepackten Katalysators erfolgt die Regenerierung vorzugsweise in der Umsetzungsvorrichtung selbst, wobei der Katalysator dazu weder aus- noch eingebaut werden muß, so dass er keinerlei zusätzlicher mechanischer Belastung unterliegt. Bei der Regenerierung des Katalysators in der Umsetzungsvorrichtung an sich wird zunächst die Umsetzung unterbrochen, gegebenenfalls vorhandenes Umsetzungsgemisch entfernt, die Regenerierung durchgeführt und anschließend die Umsetzung fortgesetzt.

Sowohl bei der Regenerierung von pulverförmigen Katalysatoren als auch bei der Regenerierung von Katalysatoren in verformter Form verläuft die erfindungsgemäße Regenerierung im wesentlichen identisch.

Insbesondere eignet sich das erfindungsgemäße Verfahren zur Regenerierung in einem Festbettreaktor, insbesondere in einem Rohr- bzw. Rohrbündelreaktor. Dabei beschreiben die Begriffe "Rohrreaktor" und "Rohrbündelreaktor" zusammengefaßte parallele Anordnungen einer Vielzahl von Kanälen in Form von Rohren, wobei die Rohre einen beliebigen Querschnitt aufweisen können. Die Rohre sind in einer festen räumlichen Beziehung zueinander angeordnet, liegen vorzugsweise voneinander räumlich beabstandet vor und sind vorzugsweise von einem Mantel umgeben, der alle Rohre umfaßt. Hierdurch kann beispielsweise ein Heiz- oder Kühlmedium durch den Mantel geführt werden, so dass alle Rohre gleichmäßig temperiert werden.

Weiter bevorzugt sind die einzelnen Rohre innerhalb des vorzugsweise verwendeten Rohr- bzw. Rohrbündelreaktors ungefähr 0,5 bis 15 m, weiter bevorzugt 5 bis 15 m, und insbesondere ungefähr 8 bis 12 m lang.

Vorzugsweise soll der Katalysator während der Regenerierung im Reaktor verbleiben. Ferner kann das erfindungsgemäße Verfahren zur Regenerierung auch auf Zeolith-Katalysatoren angewendet werden, die sich in mehreren Reaktoren, die parallel oder seriell oder (jeweils zum Teil) parallel und seriell geschaltet sind, angewendet werden.

Die erfindungsgemäße Regenerierung wird bei Temperaturen oberhalb von 120°C, vorzugsweise oberhalb von 350°C und insbesondere bei 400°C bis 650°C durchgeführt.

Bezüglich der verwendeten Regeneriergase gibt es prinzipiell keine Beschränkungen, solange die Regenerierung so geführt werden kann, dass sich der Katalysator im Inneren des Reaktors nicht derart durch zum Beispiel Abbrand der sich auf diesem befindlichen organischen Belägen, erhitzt, dass die Porenstruktur desselben und/oder der Reaktor an sich beschädigt wird, die Bildung von explosiven Gasgemischen verhindert wird und das Regeneriergas Wasserstoff enthält. Vorzugsweise wird die Regenerierung so geführt, dass sich innerhalb der Katalysatorschüttung ein Hot-Spot ausbildet, der eine Temperaturerhöhung von 10 bis 20°C, vorzugsweise nicht mehr als 20°C, bildet.

In einer besonders bevorzugten Ausführungsform umfaßt das Regeneriergas Wasserstoff oder Kohlendioxid und Wasserstoff, jeweils ggfls. noch in Kombination mit CO. Dabei beträgt der Gehalt an Wasserstoff oder CO₂ und Wasserstoff während der Regenerierung zwischen 0,1 und 100 Vol.-%, vorzugsweise 0,5 bis 20 Vol.-% und weiter bevorzugt 1 bis 5 Vol.-%.

Ferner kann das Regeneriergas weitere Inertgase, wie z.B. Stickstoff, Edelgase, wie z.B. Argon oder Helium, Kohlenwasserstoffe, wie z.B. Methan oder Ethan, und Erdgas enthalten.

Bezüglich der im Rahmen des vorliegenden Verfahrens regenerierten Zeolith-Katalysatoren existieren keine besonderen Beschränkungen.

Zeolithe sind bekanntermaßen kristalline Alumosilikate mit geordneten Kanal- und Käfigstrukturen, die Mikroporen aufweisen, die vorzugsweise kleiner als ungefähr 0,9 nm sind. Das Netzwerk solcher Zeolithe ist aufgebaut aus SiO₄- und AlO₄-Tetraedern, die über gemeinsame Sauerstoffbrücken verbunden sind. Eine Übersicht der bekannten Strukturen findet sich beispielsweise bei W. M. Meier, D. H. Olson und Ch. Baerlocher, "Atlas of Zeolithe Structure Types", Elsevier, 4. Aufl., London 1996.

Es sind nun auch Zeolithe bekannt, die kein Aluminium enthalten und bei denen im Silikatgitter an Stelle des Si(IV) teilweise Titan als Ti(IV) steht. Diese Titanzeolithe, insbesondere solche mit einer Kristallstruktur von MFI-Typ, sowie Möglichkeiten zu Ihrer Herstellung sind beschrieben, beispielsweise in der EP-A 0 311 983 oder EP-A 405 978. Außer Silicium und Titan können solche Materialien auch zusätzliche Elemente wie z. B. Aluminium, Zirkonium, Zinn, Eisen, Kobalt, Nickel, Gallium, Bor oder geringe Menge an Fluor enthalten. In den mit dem erfindungsgemäßen Verfahren vorzugsweise regenerierten Zeolith-Katalysatoren kann das Titan des Zeoliths teilweise oder vollständig durch Vanadium, Zirkonium, Chrom oder Niob oder ein Gemisch aus zwei oder mehr davon ersetzt sein. Das molare Verhältnis von Titan und/oder Vanadium, Zirkonium, Chrom oder Niob zur Summe aus Silicium und Titan und/oder Vanadium und/oder Zirkonium, und/oder Chrom und/oder Niob liegt in der Regel im Bereich von 0,01 : 1 bis 0,1 : 1.

Titanzeolithe, insbesondere solche mit einer Kristallstruktur vom MFI-Typ, sowie Möglichkeiten zu ihrer Herstellung sind beispielsweise in der WO 98/55228, EP-A 0 311 983 oder der EP-A 0 405 978 beschrieben.

Titanzeolithe mit MFI-Struktur sind dafür bekannt, dass sie über ein bestimmtes Muster bei der Bestimmung ihrer Röntgenbeugungsaufnahmen sowie zusätzlich über eine Gerüstschwingungsbande im Infrarotbereich (IR) bei etwa 960 cm⁻¹ identifiziert werden können und sich damit von Alkalimetalltitanaten oder kristallinen und amorphen TiO₂-Phasen unterscheiden.

Dabei sind im einzelnen Titan-, Germanium-, Tellur-, Vanadium-, Chrom-, Niob-, Zirkoniumhaltige Zeolithe mit Pentasil-Zeolith-Struktur, insbesondere die Typen mit röntgenografischer Zuordnung zur ABW-, ACO-, AEI-, AEL-, AEN-, AET-, AFG-, AFI-, AFN-, AFO-, AFR-, AFS-, AFT-, AFX-, AFY-, AHT-, ANA-, APC-, APD-, AST-, ATN-, ATO-, ATS-, ATT-, ATV-, AWO-, AWW-, BEA-, BIK-, BOG-, BPH-, BRE-, CAN-, CAS-, CFI-, CGF-, CGS-, CHA-, CHI-, CLO-, CON-, CZP-, DAC-, DDR-, DFO-, DFT-, DOH-, DON-, EAB-, EDI-, EMT-, EPI-, ERI-, ESV-, EUO-, FAU-, FER-, GIS-, GME-, GOO-, HEU-, IFR-, ISV-, ITE-, JBW-, KFI-, LAU-, LEV-, LIO-, LOS-, LOV-, LTA-, LTL-, LTN-, MAZ-, MEI-, MEL-, MEP-, MER-, MFI-, MFS-, MON-, MOR-, MSO-, MTF-, MTN-, MIT-, MTW-, MWW-, NAT-, NES-, NON-, OFF-, OSI-, PAR-, PAU-, PHI-, RHO-, RON-, RSN-, RTE-, RTH-, RUT-, SAO-, SAT-, SBE-, SBS-, SBT-, SFF-, SGT-, SOD-, STF-, STI-, STT-, TER-, THO-, TON-, TSC-, VET-, VFI-, VNI-, VSV-, WIE-, WEN-, YUG-, ZON-Struktur sowie zu Mischstrukturen aus zwei oder mehr der vorgenannten Strukturen zu nennen. Denkbar sind für den Einsatz im erfindungsgemäßen Verfahren weiterhin titanhaltige Zeolithe mit der Struktur des ITQ-4, SSZ-24, TTM-1, UTD-1, CIT-1 oder CIT-5. Als weitere titanhaltige Zeolithe sind solche mit der Struktur des ZSM-48 oder ZSM-12 zu nennen.

Als besonders bevorzugt sind für das erfindungsgemäße Verfahren Ti-Zeolithe mit MFI-, MEL- oder MFI/MEL-Mischstruktur anzusehen. Als weiter bevorzugt sind im einzelnen die Ti-enthaltenden Zeolith-Katalysatoren, die im allgemeinen als "TS-1", "TS-2", "TS-3" bezeichnet werden, sowie Ti-Zeolithe mit einer zu β-Zeolith isomorphen Gerüststruktur zu nennen.

Demgemäß betrifft die vorliegende Erfindung auch ein Verfahren, wie oben beschrieben, dadurch gekennzeichnet, dass der Katalysator ein Titansilikalit der Struktur TS-1 ist.

Unter dem Begriff"Alken", wie er im Rahmen der vorliegenden Erfindung verwendet wird, werden sämtliche Verbindungen verstanden, die mindestens eine C-C-Doppelbindung aufweisen.

Als Beispiele für solche organischen Verbindungen mit mindestens einer C-C-Doppelbindung seien folgende Alkene genannt:
Ethen, Propen, 1-Buten, 2-Buten, Isobuten, Butadien, Pentene, Piperylen, Hexene, Hexadiene, Heptene, Octene, Diisobuten, Trimethylpenten, Nonene, Dodecen, Tridecen, Tetra- bis Eicosene, Tri- und Tetrapropen, Polybutadiene, Polyisobutene, Isoprene, Terpene, Geraniol, Linalool, Linalylacetat, Methylencyclopropan, Cyclopenten, Cyclohexen, Norbomen, Cyclohepten, Vinylcyclohexan, Vinyloxiran, Vinylcyclohexen, Styrol, Cycloocten, Cyclooctadien, Vinylnorbornen, Inden, Tetrahydroinden, Methylstyrol, Dicyclopentadien, Divinylbenzol, Cyclododecen, Cyclododecatrien, Stilben, Diphenylbutadien, Vitamin A, Betacarotin, Vinylidenfluorid, Allylhalogenide, Crotylchlorid, Methallylchlorid, Dichlorbuten, Allylalkohol, Methallylalkohol, Butenole, Butendiole, Cyclopentendiole, Pentenole, Octadienole, Tridecenole, ungesättigte Steroide, Ethoxyethen, Isoeugenol, Anethol, ungesättigte Carbonsäuren wie z.B. Acrylsäure, Methacrylsäure, Crotonsäure, Maleinsäure, Vinylessigsäure, ungesättigte Fettsäuren, wie z.B. Ölsäure, Linolsäure, Palmitinsäure, natürlich vorkommende Fette und Öle.

Bevorzugt werden im erfindungsgemäßen Verfahren Alkene verwendet, die 2 bis 8 Kohlenstoffatome enthalten. Besonders bevorzugt werden Ethen, Propen, und Buten umgesetzt. Insbesondere bevorzugt wird Propen umgesetzt.

Demgemäß betrifft die vorliegende Erfindung auch ein Verfahren, wie oben beschrieben, oder ein integriertes Verfahren, wie oben beschrieben, dadurch gekennzeichnet, dass das Alken Propen ist.

Der Begriff "Hydroperoxid" umfaßt alle Hydroperoxide einschließlich Wasserstoffperoxid, wobei bezüglich der im Rahmen des erfindungsgemäßen Verfahrens verwendbaren Hydroperoxid-Lösungen sowie deren Herstellung auf den Stand der Technik Bezug genommen wird. Hierzu verweisen wir beispielhaft auf die DE 19723950.1 und den darin zitierten Stand der Technik.

Zur Herstellung des verwendeten Wasserstoffperoxides kann dabei beispielsweise auf das Anthrachinonverfahren zurückgegriffen werden, nach dem praktisch die gesamte Menge des weltweit produzierten Wasserstoffperoxids hergestellt wird. Dieses Verfahren beruht auf der katalytischen Hydrierung einer Anthrachinon-Verbindung zur entsprechenden Anthrahydrochinon-Verbindung, nachfolgender Umsetzung derselben mit Sauerstoff unter Bildung von Wasserstoffperoxid und anschließender Abtrennung des gebildeten Wasserstoffperoxids durch Extraktion. Der Katalysezyklus wird durch erneute Hydrierung der rückgebildeten Anthrachinon-Verbindung geschlossen.

Einen Überblick über das Anthrachinonverfahren gibt "Ullmanns Encyclopedia of Industrial Chemistry", 5. Auflage, Band 13, Seiten 447 bis 456.

Ebenso ist es denkbar, zur Wasserstoffperoxidgewinnung Schwefelsäure durch anodische Oxidation unter gleichzeitiger kathodischer Wasserstoffentwicklung in Peroxodischwefelsäure zu überführen. Die Hydrolyse der Peroxodischwefelsäure führt dann auf dem Weg über Peroxoschwefelsäure zu Wasserstoffperoxid und Schwefelsäure, die damit zurückgewonnen wird.

Möglich ist selbstverständlich auch die Darstellung von Wasserstoffperoxid aus den Elementen.

Vor dem Einsatz von Wasserstoffperoxid im erfindungsgemäßen Verfahren ist es möglich, beispielsweise eine kommerziell erhältliche Wasserstoffperoxid-Lösung von unerwünschten Ionen zu befreien. Dabei sind unter anderem Methoden denkbar, wie sie beispielsweise in der WO 98/54086, in der DE-A 42 22 109 oder in der WO 92/06918 beschrieben sind. Ebenso kann mindestens ein Salz, das in der Wasserstoffperoxid-Lösung enthalten ist, durch eine Vorrichtung aus der Wasserstoffperoxid-Lösung mittels Ionenaustausch entfernt werden, die dadurch gekennzeichnet ist, dass die Vorrichtung mindestens ein nicht-saures Ionenaustauscherbett mit einer Strömungsquerschnittsfläche F und einer Höhe H aufweist, wobei die Höhe H des Ionenaustauscherbettes kleiner oder gleich 2,5 • F^{1/2} und insbesondere kleiner oder gleich 1,5 • F^{1/2} ist. Im Rahmen der vorliegenden Erfindung können prinzipiell alle nicht-sauren Ionenaustauscherbetten mit Kationenaustauscher und/oder Anionenaustauscher eingesetzt werden. Auch innerhalb eines Ionenaustauscherbettes können Kationen- und Anionenaustauscher als sogenannte Mischbetten verwendet werden. In einer bevorzugten Ausführungsform der vorliegenden Erfindung wird nur ein Typ von nicht-saueren Ionenaustauschern eingesetzt. Weiter bevorzugt ist der Einsatz eines basischen Ionentauschers, besonders bevorzugt der eines basischen Anionentauschers und weiter besonders bevorzugt der eines schwach basischen Anionentauschers.

In einer besonders bevorzugten Ausführungsform betrifft die vorliegende Erfindung ein Verfahren zur Oxidation eines Alkens, das die folgenden Stufen (1) bis (4) umfaßt:
(1) Waschen des Zeolith-Katalysators mit einem Lösungsmittel
(2) Trocknen des gewaschenen Zeolith-Katalysators bei einer Temperatur von-50 bis 250 °C
(3) Aufheizen des getrockneten Katalysators
(4) Regenerieren des aufgeheizten Katalysators mittels eines Verfahrens gemäß der vorliegenden Erfindung.

Weiter bevorzugt umfaßt dieses bevorzugte Verfahren die weiteren Stufen (5) und/oder (6):
(5) Abkühlen des gemäß Stufe (4) erhaltenen regenerierten Katalysators
(6) Konditionieren des gemäß Stufe (4) oder gemäß Stufe (5) erhaltenen Katalysators.

Diese Stufen werden nunmehr nochmals einzeln detailliert beschrieben. Dabei ist zunächst zu beachten, dass es sich bei dem zu regenerierenden Zeolith-Katalysator um einen Katalysator handelt, der bei einer Oxidation eines Alkens durch Umsetzung des Alkens mit einem Hydroperoxid, vorzugsweise einer Umsetzung, die kontinuierlich durchgeführt wurde, eingesetzt wird und infolge eines Aktivitätsabfalls nunmehr regeneriert werden muß. Dabei wird, wie oben bereits angedeutet, die erfindungsgemäße Regenerierung vorzugsweise in dem oder den Reaktor(en) durchgeführt, in dem (denen) auch die Umsetzung des Alkens mit einem Hydroperoxids in Gegenwart des zu regenerierenden Katalysators durchgeführt wird.

In einer weiteren, ganz besonders bevorzugten Ausführungsform wird dabei der Reaktor im Verbund mit der Aufarbeitung des Wertprodukts sowie der erfindungsgemäßen Regenerierung betrieben, da diese Fahrweise einen geschlossenen Kreislauf an Lösungsmittel erlaubt.

### (1) Waschen des Zeolith-Katalysators mit einem Lösungsmittel

Der erste Schritt dieser Ausführungsform der erfindungsgemäßen Regenerierung beinhaltet zunächst das Waschen des desaktivierten Katalysators mit einem Lösungsmittel. Dafür wird zunächst die Zufuhr der Einsatzstoffe von Hydroperoxid und organischer Verbindung unterbrochen. Als Lösungsmittel können dabei alle Lösungsmittel eingesetzt werden, in denen sich das jeweilige Umsetzungsprodukt der Oxidation des Alkens gut löst. Vorzugsweise werden derartige Lösungsmittel ausgewählt aus der Gruppe, bestehend aus Wasser, einem Alkohol, vorzugsweise Methanol, einem Aldehyd, einer Säure, wie z.B. Ameisensäure, Essigsäure oder Propionsäure, einem Nitril, einem Kohlenwasserstoff, einem halogeniertem Kohlenwasserstoff, verwendet. Bezüglich Details zu derartigen Lösungsmitteln wird Bezug genommen auf die WO 98/55228.

Bevorzugt werden Lösungsmittel, die schon bei der Epoxidierung von Olefin unter Verwendung des zu regenerierenden Katalysators als Lösungsmittel fungieren, eingesetzt. Als solche sind beispielhaft zu nennen: Wasser, Alkohole, wie z.B. Methanol, Ethanol, 1-Propanol, 2-Propanol, 2-Methyl-2-propanol, 1-Butanol, 2-Butanol, Allylalkohol oder Ethylenglycol, oder Ketone, wie z.B. Aceton, 2-Butanon, 2-Methyl-3-butanon, 2-Pentanon, 3-Pentanon, 2-Methyl-4-pentanon oder Cyclohexanon.

Sofern als Lösungsmittel für das Waschen das bereits in der Umsetzung verwendete Lösungsmittel eingesetzt wird, wird dessen Zufuhr weitergeführt und der Katalysator bei einer Temperatur von im allgemeinen 40 bis 200 °C, ggf. mit ansteigender Temperatur und unter Druck mit dem Lösungsmittel gewaschen. Dabei wird das Waschen vorzugsweise solange fortgesetzt, bis der Gehalt an Umsetzungsprodukt im Austrag unter 1% des Anfangswertes sinkt. Sofern ein anderes Lösungsmittel verwendet werden soll, wird nach der Reaktion die Zufuhr von Hydroperoxid, dem Umsetzungsprodukt und dem Lösungsmittel der Reaktion unterbrochen und die Zufuhr des Lösungsmittels zum Waschen gestartet. Besonders bevorzugt ist die Verwendung desselben Lösungsmittels für die Reaktion und für das Waschen des Katalysators.

Bezüglich der Dauer des Waschvorgangs gibt es keinerlei Beschränkungen, wobei längere Waschzeiten und damit eine möglichst weitgehende Entfernung des Umsetzungsprodukts bzw. der organischen Beläge von Vorteil ist.

### (2) Trocknen des gewaschenen Zeolith-Katalysators bei einer Temperatur von -50 bis 250 °C

Nach dem Waschen des Katalysators wird das verwendete Lösungsmittel aus dem Reaktor abgelassen bzw. abgepumpt. Der poröse Katalysator enthält dann noch erhebliche Mengen an anhaftendem Lösungsmittel, das durch Trocknen mit einem Gasstrom bei Temperaturen von -50 bis 250 °C, wobei die verwendete Temperatur in der Nähe des Siedepunktes des Lösungsmittels bei dem jeweils gewünschten Druck liegt, zum überwiegenden Teil entfernt wird. Dabei liegen die Temperaturen typischerweise im Bereich von ± 50°C ober- oder unterhalb des Siedepunktes.

Zum Trocknen wird im allgemeinen ein Inertgas, wie z.B. Stickstoff, Argon, CO₂, Wasserstoff, Synthesegas, Methan, Ethan oder Erdgas verwendet. Vorzugsweise wird Stickstoff eingesetzt. Das mit Lösungsmittel beladene Gas wird dann entweder entsorgt, beispielsweise durch Verbrennung mit einer Fackel, oder an einer geeigneten Stelle, beispielsweise bei der Aufarbeitung des Umsetzungsprodukts aus dem Verfahren zur Oxidation eines Alkens, eingespeist und das darin enthaltene Lösungsmittel zurückgewonnen.

In einer bevorzugten Ausführungsform wird das Waschen unter Druck bei einer Temperatur oberhalb des Siedepunkts des Lösungsmittels durchgeführt und nach Ablassen des Lösungsmittels der Druck soweit abgesenkt, dass bereits vor oder während der Zufuhr von Gas für die Trocknung durch die Latentwärme des Reaktors schon ein Teil des Lösungsmittels verdampft.

Für die Zufuhr von Wärme innerhalb dieses Schritts kann sowohl ein Gas als auch eine Flüssigkeit, zum Beispiel innerhalb des Mantels eines Rohrreaktors, eingesetzt werden. Bevorzugt ist die Verwendung von einer Flüssigkeit für den Temperaturbereich unter 150 °C und von einem Gas für den Temperaturbereich oberhalb 150 °C.

### (3) Aufheizen des getrockneten Katalysators

Nach dem Trocknen wird der zu regenerierende Katalysator aufgeheizt. Dieses Aufheizen kann nach allen dem Fachmann geläufigen Methoden durchgeführt werden, wobei die Aufheizung vorzugsweise in Gegenwart eines Inertgasstroms, wie z.B. Stickstoff, CO₂, Argon, Methan, Ethan oder Erdgas, durchgeführt wird.

In einer besonderen Ausführungsform des erfindungsgemäßen Verfahrens befindet sich der Katalysator in den Rohren eines Rohrbündelreaktors. In derartigen Reaktoren wird die Wärme durch den Mantelraum in das System eingetragen. Dabei muß die Heizrate so gewählt werden, dass im Reaktor keine unzulässig hohen mechanischen Spannungen entstehen. Typische Heizraten betragen 0,01 °C/min bis 0,2 °C/min.

### (4) Regenerieren des aufgeheizten Katalysators mittels eines Verfahrens gemäß der vorliegenden Erfindung

Anschließend wird die Regenerierung des Katalysators, wie im Rahmen der vorliegenden Anmeldung ausführlich beschrieben, durchgeführt.

### (5) Abkühlen des gemäß Stufe (4) erhaltenen regenerierten Katalysators

Nach Beendigung der Regenerierung gemäß Schritt (4) kann der regenerierte Katalysator, vorzugsweise der gesamte Reaktor mit dem sich darin befindlichen regenerierten Katalysator, auf eine Temperatur von vorzugsweise unterhalb 200 °C abgekühlt werden.

### (6) Konditionieren des gemäß Stufe (4) oder Stufe (5) erhaltenen Katalysators

Nach der erfindungsgemäßen Regenerierung bzw. dem Abkühlen kann der Katalysator noch konditioniert werden, um die Sorptionswärme des Lösungsmittels bzw. der Edukte vor dem erneuten Einsatz des Katalysators kontrolliert abzuführen. Dazu werden dem am Katalysator vorbeiströmenden Inertgas geringe Mengen eines Lösungsmittels, vorzugsweise des gleichen Lösungsmittels, welches für die Reaktion oder für die Wäsche des Katalysators eingesetzt worden ist, insbesondere ein Alkohol, wie z.B. Methanol, zugemischt und der Lösungsmitteldampf-haltige Inertgasstrom durch das Katalysatorbett geleitet. Der Lösungsmittelgehalt und der Volumenstrom des Konditioniergases werden so gewählt, dass am Katalysator keine unzulässige Spitzentemperatur auftritt. Bevorzugt soll die Temperaturerhöhung nicht mehr als 100 °C über der mittleren Temperatur des Wärmeüberträgers, z.B. im Mantelraum eines Rohrreaktors, liegen.

Nach dem Abklingen der Wärmefreisetzung wird die Zufuhr an Konditioniergas mit Lösungsmittel unterbrochen und der Reaktor, vorzugsweise der Festbettreaktor, flüssig gefüllt und wieder in Betrieb genommen.

Bei den optionalen Stufen (5) und (6) des erfindungsgemäßen Verfahrens ist es wichtig, dass sowohl das Abkühlen nicht zu schnell durchgeführt wird, als auch dass die Konditionierung nicht zu schnell durchgeführt wird, da beide Vorgänge die Schüttung des Katalysators im Reaktor negativ beeinflussen können. Darüber hinaus sollte auch ein zu rascher Temperaturanstieg innerhalb des Katalysators bei der Konditionierung aus den gleichen Gründen vermieden werden.

Der regenerierte Kalalysator wird wie oben dargelegt, wieder zur Umsetzung des Alkens mit dem Hydroperoxid eingesetzt. Insbesondere läßt sich das erfindungsgemäße Verfahren zur Oxidation eines Alkens für die Umsetzung von Propylen zu Propylenoxid mittels Wasserstoffperoxid, weiter bevorzugt in methanolischer Lösung, anwenden.

Das erfindungsgemäße Verfahren weist insbesondere folgende Vorteile auf:
- Durch die schonende Reaktionsführung gelingt es, Zeolith-Katalysatoren derart zu regenerieren, dass die Aktivität nach Regenerierung weitgehend erhalten bleibt;
- das erfindungsgemäße Regenerierverfahren kann, bei Verwendung eines Festbettkatalysators, ohne Ausbau des Katalysators im Reaktor selbst durchgeführt werden;
- die im Rahmen des erfindungsgemäßen Regenerierverfahrens eingesetzten Lösungsmittel können identisch mit den Lösungsmitteln während der Umsetzung sein und insgesamt vollständig im Kreis geführt werden.

Die Erfindung soll nunmehr noch anhand einiger erfindungsgemäßer Beispiele näher erläutert werden.

### Beispiel

Ein in der Epoxidation von Propen zu Propylenoxid eingesetzter Heterogenkatalysator (60 Gew.-% Titanzeolith TS-1, 40 Gew.-% SiO₂-Binder) wurde nach einer Laufzeit von mehreren hundert Stunden (850 h) aus einem Festbett-Röhrenreaktor ausgebaut. Das Material enthielt nach der chemischen Analyse einen Kohlenstoffgehalt von 1,2 Gew.-%. In einem Standard-Aktivitätstest gemäß DE 198 59 561.1, Beispiel 10, erbrachte dieser Katalysator nur noch einen Gehalt an Propylenoxid von 3,6 Gew.-%.

In einem Rohrreaktor (2000 mm Länge, Innendurchmesser 40 mm) wurden 800 g dieses Materials innerhalb von 2 Stunden in einem Strom aus 900 1/h Stickstoff und 100 1/h Wasserstoff auf 450 °C aufgeheizt, 6 Stunden bei diesen Bedingungen gehalten und wieder abgekühlt. Es wurde keine Exothermie im Reaktor beobachtet.

Der regenerierte Katalysator hatte nur noch einen Kohlenstoffgehalt von 0,1 Gew.-%. Im oben definierten Standard-Aktivitätstest erbrachte dieser Katalysator einen Gehalt an Propylenoxid von 4,9 Gew.-%.

## Patentansprüche

1. Verfahren zur Oxidation eines Alkens, das umfasst:
Umsetzung des Alkens mit einem Hydroperoxid in Gegenwart eines Zeolith-Katalysators und anschließende Regenerierung des Katalysators mittels eines Verfahrens, das eine thermische Behandlung des Katalysators in Gegenwart eines Gasstroms bei Temperaturen oberhalb von 120°C umfasst, wobei der Gasstrom Wasserstoff enthält, und wobei der regenerierte Katalysator wieder zur Umsetzung des Alkens mit dem Hydroperoxid eingesetzt wird.

2. Verfahren nach Anspruch 1, wobei der Gasstrom neben Wasserstoff CO₂ und/oder CO umfasst.

3. Verfahren nach Anspruch 1 oder 2, wobei der Zeolith-Katalysator ein Titansilikalit mit MFI-Struktur ist.

4. Verfahren nach einem der Ansprüche 1 bis 3, wobei die Regenerierung in einem Festbettreaktor durchgeführt wird.

5. Verfahren nach Anspruch 4, wobei der Festbettreaktor ein Rohr- oder Rohrbündelreaktor ist.

6. Verfahren nach einem der Ansprüche 1 bis 5, das die folgenden Stufen umfasst:
(1) Waschen des Zeolith-Katalysators mit einem Lösungsmittel
(2) Trocknen des gewaschenen Zeolith-Katalysators bei einer Temperatur von -50 bis 250 °C
(3) Aufheizen des getrockneten Katalysators
(4) Regenerieren des aufgeheizten Katalysators mittels eines Verfahrens nach einem der Ansprüche 1 bis 5.

7. Verfahren nach Anspruch 6, das weiterhin mindestens eine der folgenden Stufen (5) und (6) umfaßt, die nach der Stufe (4) durchgeführt werden:
(5) Abkühlen des gemäß Stufe (4) erhaltenen regenerierten Katalysators
(6) Konditionieren des gemäß Stufe (4) oder gemäß Stufe (5) erhaltenen Katalysators, wobei dem am Katalysator vorbeiströmendem Inertgas geringe Mengen eines Lösungsmittels zugemischt werden und der Lösungsmitteldampf-haltige Inertgasstrom durch das Katalysatorbett geleitet wird.

8. Verfahren nach einem der Ansprüche 1 bis 7, wobei das Alken Propen ist und das Hydroperoxid Wasserstoffperoxid ist.

## Claims

1. A process for the oxidation of an alkene, which comprises:
reaction of the alkene with a hydroperoxide in the presence of a zeolite catalyst and subsequent regeneration of the catalyst by means of a process which comprises thermal treatment of the catalyst at above 120°C in the presence of a gas stream comprising hydrogen, wherein the regenerated catalyst is reused for the reaction of the alkene with the hydroperoxide.

2. A process as claimed in claim 1, wherein the gas stream further comprises CO₂ and/or CO in addition to hydrogen.

3. A process as claimed in claim 1 or 2, wherein the zeolite catalyst is a titanium silicalite having an MFI structure.

4. A process as claimed in any of claims 1 to 3, wherein the regeneration is carried out in a fixed-bed reactor.

5. A process as claimed in claim 4, wherein the fixed-bed reactor is a tube reactor or a shell-and-tube reactor.

6. A process as claimed in any of claims 1 to 5 which comprises the following steps:
(1) washing the zeolite catalyst with a solvent
(2) drying the washed zeolite catalyst at from - 50 to 250°C
(3) heating the dried catalyst
(4) regenerating the heated catalyst by means of a process as claimed in any of claims 1 to 5.

7. A process as claimed in claim 6 which further comprises at least one of steps (5) and (6) below, which are carried out after step (4):
(5) cooling the regenerated catalyst obtained in step (4)
(6) conditioning the catalyst obtained in step (4) or in step (5), wherein small amounts of a solvent are mixed into the inert gas flowing past the catalyst and the inert gas stream laden with solvent vapor is passed through the catalyst bed.

8. A process as claimed in any of claims 1 to 7, wherein the alkene is propene and the hydroperoxide is hydrogen peroxide.

## Revendications

1. Procédé d'oxydation d'un alcène, qui comprend :
la réaction de l'alcène avec un hydroperoxyde en présence d'un catalyseur à zéolithe, suivie par la régénération du catalyseur au moyen d'un procédé, qui comprend un traitement thermique du catalyseur en présence d'un courant gazeux à des températures supérieures à 120°C, le courant gazeux contenant de l'hydrogène, et le catalyseur régénéré étant de nouveau mis en oeuvre pour la réaction de l'alcène avec l'hydroperoxyde.

2. Procédé selon la revendication 1, dans lequel le courant gazeux contient outre l'hydrogène, du CO₂ et/ou du CO.

3. Procédé selon la revendication 1 ou 2, dans lequel le catalyseur à zéolithe est une silicalite de titane ayant une structure MFI.

4. Procédé selon l'une quelconque des revendications 1 à 3, dans lequel la régénération est réalisée dans un réacteur à lit fixe.

5. Procédé selon la revendication 4, dans lequel le réacteur à lit fixe est un réacteur tubulaire ou à faisceau tubulaire.

6. Procédé selon l'une quelconque des revendications 1 à 5, qui comprend les étapes suivantes :
(1) lavage du catalyseur à zéolithe par un solvant,
(2) séchage du catalyseur à zéolithe lavé à une température de -50 à 250°C,
(3) chauffage du catalyseur séché,
(4) régénération du catalyseur chauffé au moyen d'un procédé selon l'une quelconque des revendications 1 à 5.

7. Procédé selon la revendication 6, qui comprend en outre au moins l'une des étapes (5) et (6) suivantes, qui sont réalisées après l'étape (4) :
(5) refroidissement du catalyseur régénéré obtenu selon l'étape (4)
(6) conditionnement du catalyseur obtenu selon l'étape (4) ou selon l'étape (5), de faibles quantités d'un solvant étant mélangées au gaz inerte s'écoulant au voisinage du catalyseur et le courant de gaz inerte contenant des vapeurs de solvant étant conduit à travers le lit de catalyseur.

8. Procédé selon l'une quelconque des revendications 1 à 7, dans lequel l'alcène est le propène et l'hydroperoxyde est le peroxyde d'hydrogène.
